Europäisches Patentamt

European Patent Office

Office européen des brevets

(11) Publication number: **0 207 770**
**A1**

# (12) EUROPEAN PATENT APPLICATION

(21) Application number: 86305055.5

(22) Date of filing: 27.06.86

(51) Int. Cl.⁴: **C 12 N 15/00**

(30) Priority: 03.07.85 US 751697

(43) Date of publication of application:
07.01.87 Bulletin 87/2

(84) Designated Contracting States:
AT CH DE FR GB IT LI

(71) Applicant: SCHERING CORPORATION
2000 Galloping Hill Road
Kenilworth, New Jersey 07033(US)

(72) Inventor: Finkelstein, Mark
30 Elberta Road
Maplewood New Jersey 07040(US)

(72) Inventor: Leibowitz, Paul Jason
185 Prospect Avenue Apt. 2N
Hackensack New Jersey 07601(US)

(74) Representative: Ritter, Stephen David et al,
Mathys & Squire 10 Fleet Street
London EC4Y 1AY(GB)

(54) Thermoinducible plasmid.

(57) A novel thermoinducible chimeric plasmid designated pKG-2 is disclosed as a preferred embodiment of plasmids derived from pBR322 but with the replicon exchanged for a thermoinducible replicon.

EP 0 207 770 A1

Croydon Printing Company Ltd.

## THERMOINDUCIBLE PLASMID

This invention relates to novel thermoinducible chimeric plasmids, especially plasmids derived from pBR322 but with the replicon exchanged for a thermoinducible replicon, and in particular to one such plasmid designated pKG-2.

Plasmid pKG-2 is constructed by replacing a portion of miniplasmid pVU208 derived from parent CloDF-13 with a portion of the well-known pBR322 plasmid.

The usefulness of plasmids as cloning vectors in genetic engineering is well established, as is the preparation of new plasmids by combining portions of known plasmids containing desirable characteristics. Also, the use of thermoinducible or "run-away" plasmids for overproduction of gene products is well documented; see for example Uhlin, B.E., et al., Gene, 6, (1979) pp. 91-106, and U.S. Patents 4,487,835, 4,495,287 and 4,499,189.

pBR322 is one of the most commonly used plasmid vectors; its entire nucleotide sequence is known (see Sutcliffe, J.G., Cold Spring Harb. Symp. Quant. Biol., 43 (1978) pp. 77-90); it has resistance to both ampicillin and tetracycline.

-2-

The invention provides a runaway plasmid comprising a substantial part of the DNA sequence of plasmid pBR322 but lacking the replicon thereof, and containing a thermoinducible replicon. The novel plasmid has a controlled copy number at relatively low temperatures, e.g. around 30°C, but an unregulated copy number at higher temperatures, e.g. it starts to show runaway behavior at 34-35°C and shows marked runaway behavior at 37-40°C.

This plasmid is stable under replication conditions, i.e. it is not lost.

Whereas most of the research directed at thermoinducible plasmids has been based on plasmids derived from R1, a substantial effort has been directed at plasmids derived from CloDF-13; see for example Nijkamp, H.J.J., et al., Molecular Biology, Pathogenicity and Ecology of Bacterial Plasmids, ed. Levy, S. (New York: Plenum Publishers, 1981) pp. 247-258, and Stuitje, A.R., et al., Nature, 290 (1981) pp. 264-267. CloDF-13 was originally isolated from Enterobacter cloacae, but is maintained in Escherichia coli (Veltkamp, E., et al., B.B.A., 425 (1976) pp. 356-367), and shares many properties of the $ColE_1$-type plasmid. Since it is non-conjugative and poorly mobilized, CloDF-13 is well suited for use as a cloning vector. The complete nucleotide sequence of the region essential for plasmid replication has been published and a series of specific mutants displaying thermosensitive plasmid copy numbers has been described; see Stuitje et al., supra.

The CloDF-13 miniplasmid pVU208 used as the starting replicon (Hakkaart, M.J., et al., Mol. Gen. Genet., 183 (1981) pp. 326-332) contains an integrated ampicillin transposon (Tn 901), is Clo-, Imm-, and mitomycin C sensitive, and displays a copl temperature-sensitive phenotype as a result of a G$\rightarrow$A base

transition at position 205 bp downstream from the Bam HI site. This copl phenotype allows for a modest plasmid copy number at about 30°C and a greatly elevated plasmid copy number at temperatures above about 35°C.

The plasmid of the present invention preferably has such a modified Clo replicon as thermoinducible replicon, in particular the replicon of plasmid pVU208. (The Clo replicon is derived from a cloacin plasmid such as CloDF-13.)

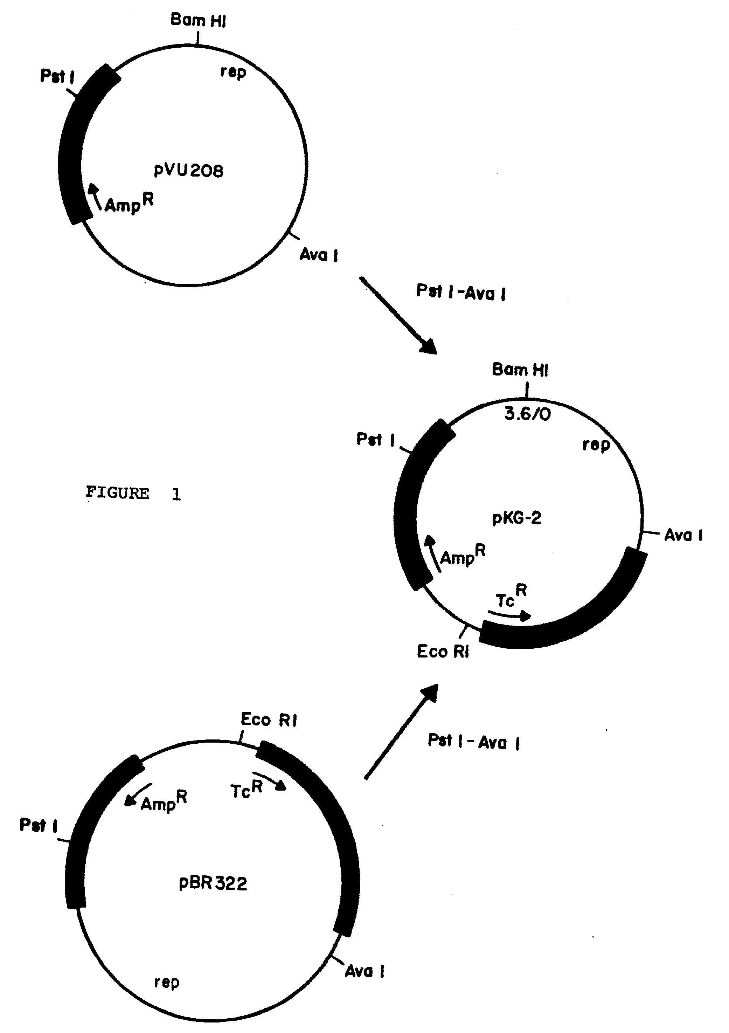

A preferred embodiment of the present invention will now be described in conjunction with the single Figure 1 in the accompanying drawing, which illustrates the construction of pKG-2 by reference to restriction endonuclease cleavage maps of pBR322 and pVU208. $Amp^R$ and $Tc^R$ refer to genes conferring resistance to ampicillin and tetracycline respectively, and rep refers to origin of vegetative replication; the restriction endonuclease cleavage sites indicated are described below.

Plasmid pKG-2 is constructed as shown in Figure 1 by replacing approximately 1.9 Kb of the non-essential region of pVU208 plasmid with the 2.2 Kb PstI-AvaI fragment from pBR322 containing the tetracycline resistance operon and that part of the β-lactamase gene required to reconstitute ampicillin resistance. In other words, an approximately 1.4 Kb PstI-AvaI fragment of PVU208 (including replicon) is ligated with the approximately 2.2 Kb PstI-AvaI fragment of pBR322 (without replicon). This newly constructed 3.6 Kb plasmid displays all of the thermoinducible properties of the parent replicon as well as an array of unique cloning and insertion inactivation sites found in pBR322. Plasmid pKG-2 is remarkably stable and replicates in a predictable manner in a given bacterial host.

-4-

Unique cleavage sites for restriction endonucleases in pKG-2 include the following:

| Restriction enzyme | Number of Cleavage Sites |
|---|---|
| Bam HI | 1 |
| PstI | 1 |
| EcoRI | 1 |
| Ava I | 1 |

Bam HI is isolated from Bacillus amyloliquefaciens, PstI from Providencia stuartii, EcoRI from Escherichia coli, and Ava I from Anabaena variabilis.

Plasmid pKG-2 was deposited with the American Type Culture Collection (ATCC) in Rockville, Md., U.S.A. on June 13, 1985, under deposit number ATCC 53148.

A multicopy plasmid such as pKG-2 can be used to make a bacterial cell overproduce a given gene product by cloning a foreign DNA fragment containing the gene of interest into the plasmid (together with an appropriate regulatory sequence) and transforming the plasmid into the host (i.e. bacterial) cell. Using conventional techniques, the host cell is cultured and the desired gene product is harvested. Specifically, the pKG-2 plasmid has been used as a vector into which a number of important eucaryotic genes have been inserted to augment the production of eucaryotic genes or gene products, e.g. proteins, in a bacterium (specifically Escherichia coli (E. coli)). For example, Table 1 shows the increase in production obtained when several interferon genes with their appropriate bacterial promoters, ribosome binding sites and 3' non-coding tail regions were excised from their pBR322-derived parents and introduced into pKG-2.

-5-

In each case a 12-50-fold increase in interferon titer was observed over that of the parental construction. This over-production was due solely to the increased plasmid copy number associated with the runaway phenotype, since the bacterial host, media, length of the fermentation, final cell densities and the regulatory region of the interferon operon were constant.

TABLE 1

### Overproduction of Various α-Interferons in Bacterial Host Strain Cl-31[1]

| Plasmid | Specific Interferon | Growth Temperature (°C) | Relative[2] Activity |
|---|---|---|---|
| pBR322 | alpha-2 | 37 | 1 |
| pKG-2 | alpha-2 | 30-41-30[3] | 50 |
| pBR322 | alpha-4 | 37 | 1 |
| pKG-2 | alpha-4 | 30-41-30[3] | 12 |
| pBR322 | alpha-5 | 37 | 1 |
| pKG-2 | alpha-5 | 30-41-30[3] | 32 |

(1) Bacterial host strain Cl-31 is a mini-cell producer derived from E. coli K-12 DS-410 (Dougan, G., and Sherratt, D., Molec. Gen. Genet., 151 (1977), pp. 151-160).

(2) Relative activity was determined by the cytopathic effect inhibition assay employing EMC virus and human foreskin cells. Using NIH/WHO natural leukocyte interferon as a standard, the assay was performed essentially as described by Familletti, P.C., et al., in "A Convenient and Rapid Cytopathic Effect Inhibition Assay for Interferon," Methods in Enzymology, Peska, S., ed. (New York: Academic Press, 1980), pp. 387-394.

(3) Growing culture was shifted from 30°C to 41°C for a specified time period, e.g. about 2 hours, and then downshifted from 41°C to 30°C for another specified time period, e.g. 2-3 hours. In each instance the growth medium consisted of 20 g Tryptone, 10 g Yeast Extract, 5 g NaCl, and 10 mg tetracycline per liter.

The following polypeptides inter alia have also been overproduced by plasmid pKG-2 or derivatives thereof in a variety of E. coli hosts:

murine interleukin-2; murine interleukin-3; murine and human G/M-CSF (granulocyte macrophage colony stimulating factor); and human γ-interferon-A.

Plasmid pKG-2 is constructed by techniques well known in molecular biology. Strains of the starting plasmids, pVU208 and pBR322, are grown and plasmid DNA is isolated by conventional techniques, then recombined by known methods, e.g. by digestion of each plasmid with appropriate restriction enzymes, separation of each resultant mixture, isolation of each desired fragment, ligation of these fragments, transformation into suitable host cells and finally growth and isolation of the pKG-2 plasmid.

Those skilled in the art will recognize that there are many methods for preparing plasmids. An example of such a method for the preparation of the plasmid of this invention, pKG-2, is as follows: Each starting plasmid is grown overnight in E. coli at 30°C at 300 rpm on a rotary shaker in a medium known as 20-10-5 which comprises 20 gm Tryptone, 10 gm yeast extract and 5 gm of sodium chloride per liter. The resultant plasmids are isolated from stationary cultures by the cleared lysate method followed by Cesium Chloride-Ethidium Bromide buoyant-density centrifugation; the ethidium bromide is removed from the plasmid with cesium chloride-saturated isopropanol, and the plasmid is precipitated twice in ethanol and resuspended in pH8 TE buffer (i.e. 10 mM tris(hydroxymethyl)aminomethane/HCl, 0.5 mM ethylenediamine tetraacetic acid) at 1 mg/1 ml.

pVU208 plasmid is then digested with PstI and Ava I restriction endonucleases and the resultant material electrophoresed on a 0.7% agarose gel. The approximately 1.4 kb fragment containing the runaway origin of replication and part of the ampicillin gene is excised, eluted by electrophoresis, precipitated with ethanol and retained. The pBR322 plasmid is similarly treated to isolate the 2.2 kb fragment containing the tetracycline gene and the other part of the ampicillin gene. The isolated fragments are ligated in the presence of T4 DNA ligase according to well-known procedures, and the ligation mixture transformed into E. coli 294 using the calcium chloride procedure described by Dagert and Ehrlich (Gene, 6 (1979) p. 23-28). The transformation mixture is plated into 20-10-5 agar plates containing tetracycline (10 mcg/ml) and ampicillin (40 mcg/ml). Using conventional techniques, the resultant colonies are miniprepped (prepared on a small scale) and the plasmid with the desired construction, i.e. the structure of pKG-2, is isolated.

Additional methods for the isolation, purification, transformation and selection of plasmids are described in many publications, e.g. T. Maniatis et al., Molecular Cloning (Cold Spring Harbor: Cold Spring Harbor Laboratory, 1982).

0207770

-9-

CLAIMS:

1. A runaway plasmid comprising a substantial part of the DNA sequence of plasmid pBR322 but lacking the replicon thereof, and containing a thermoinducible replicon.

2. A plasmid as claimed in claim 1 wherein the thermoinducible replicon is a modified Clo replicon, preferably the replicon of plasmid pVU208.

3. A plasmid as claimed in claim 2 designated pKG-2, having a molecular size of about 3.6 kilobases (Kb), and comprising the approximately 1.4 Kb PstI-AvaI fragment of pVU208 (including replicon) and the approximately 2.2 Kb PstI-AvaI fragment of pBR322 (without replicon).

4. A recombinant plasmid containing the DNA sequence of a plasmid as claimed in any of claims 1 to 3 and, inserted at a restriction site thereof, a foreign DNA fragment coding for a polypeptide and preferably a regulatory sequence.

5. A method for producing plasmid pKG-2 which comprises cultivating a plasmid pKG-2-harboring bacterium, preferably Escherichia coli, in a culture medium, harvesting the cells therefrom and recovering plasmid pKG-2 from the cells.

6. A process for increasing the production of gene products comprising cloning a foreign DNA fragment containing the gene of interest into plasmid pKG-2, transforming the plasmid into the host cell, culturing the host cell and harvesting the gene product.

7. A method for preparing a plasmid as claimed in claim 1 which comprises deleting the replicon of plasmid pBR322 and inserting therefor a thermoinducible replicon, especially the Clo replicon, in particular the replicon of plasmid pVU208.

8. A method as claimed in claim 7 for preparing plasmid pKG-2, which comprises cleaving plasmids pBR322 and pVU208 with PstI and AvaI and ligating the approximately 2.2 Kb fragment of pBR322 to the approximately 1.4 Kb fragment of pVU208.

0207770

FIGURE 1

**European Patent Office**

**EUROPEAN SEARCH REPORT**

EP 86 30 5055

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | EP-A-0 105 554 (NEDERLANDSE CENTRALE ORGANISATIE VOOR TOEGEPAST-NATUURWETENSCHAPPELIJK ONDERZOEK) <br> * Whole document * <br><br> ----- | 1-8 | C 12 N 15/00 |
| | | | **TECHNICAL FIELDS SEARCHED (Int. Cl 4)** |
| | | | C 12 N |

The present search report has been drawn up for all claims

| Place of search | Date of completion of the search | Examiner |
|---|---|---|
| THE HAGUE | 10-09-1986 | GANEFF J.M.A. |